# EUROPEAN PATENT APPLICATION

(11) **EP 4 835 057 A1**
(43) Date of publication of application: **04.11.2026**
(21) Application number: 24912808.3
(22) Date of filing: 24.12.2024
(51) Int. Cl.: C12N 5/0783, C07K 14/725, C07K 16/28, C07K 19/00, C12N 5/10, C12N 15/12, C12N 15/13, C12N 15/62

(54) **METHOD FOR CULTURING T CELLS**

(30) Priority: 27.12.2023 JP 2023221979
(71) Applicant: AGC Inc., Chiyoda-ku Tokyo 100-8405 (JP); Kyoto Prefectural Public University Corporation, Kyoto 602-8566 (JP); Shinshu University, Matsumoto-shi, Nagano 390-8621 (JP)
(72) Inventor: KISHIMOTO, Megumi, Tokyo 100-8405 (JP); SHIMIZU, Yuto, Tokyo 100-8405 (JP); SATO, Yushi, Tokyo 100-8405 (JP); TAKEDA, Shigenori, Tokyo 100-8405 (JP); YAGYU, Shigeki, Kyoto-shi, Kyoto 602-8566 (JP); NAKAZAWA, Yozo, Matsumoto-shi, Nagano 390-8621 (JP); TANAKA, Miyuki, Matsumoto-shi, Nagano 390-8621 (JP)
(74) Representative: TBK
(86) International application number: PCT/JP2024/045680
(87) International publication number: WO 2025/142921

(57) **Abstract**

The present invention relates to a method for culturing a T cell, including a step of contacting a composition comprising T cells with a stimulant, wherein
the stimulant activates (i) one or more intracellular signaling domains of one or more components of a TCR complex and (ii) one or more intracellular signaling domains of one or more co-stimulatory molecules. The method makes it possible to maintain and proliferate T cells in a composition containing T cells into a cell population suitable for gene transfer.

## Description

### [Technical Field]

The present invention relates to a method for culturing T cells, particularly, a method for culturing T cells in a composition containing T cells that serve as raw materials for producing T cells (T cell preparations) for use in T cell therapy.

### [Background Art]

As an immunotherapy for cancer patients, T cell therapy is known. In T-cell therapy, T cells are collected from the body, activated/amplified, and then returned. For example, methods using tumor-infiltrating lymphocytes (TIL) (Non Patent Literature 1), and methods introducing cancer antigen-specific T cell receptor (TCR) genes into T cells in the peripheral blood of patients (Non Patent Literature 2) are known.

In recent years, a treatment method using Chimeric Antigen Receptor (CAR) T cells (hereinafter also simply referred to as CAR-T cells) has been developed, in which T cell receptor (TCR) expressed by cytotoxic T cells (CTL) is genetically modified, and CTL thus directly and selectively recognizes tumor cells and exerts an antitumor effect (Non Patent Literature 3). That is, CAR-T cell is an artificially produced T cell obtained by introducing CAR through genetic manipulation. Cancer treatment using CAR-T cells kills cancer cells through a mechanism different from that of conventional anticancer agents and radiation therapy. Therefore, it is also expected to be effective for cancers that are intractable or resistant to treatment. CAR-T cells have already been formulated for blood tumors such as some leukemias and malignant lymphomas.

While autologous T cells and allogeneic T cells are used in the production of T cell preparations (activated T cells, CAR-T cells, etc.), autologous T cells collected from the peripheral blood of patients by apheresis, etc. are used in the production of CAR-T cells. When CAR-T cells are produced, T cells are isolated, the isolated T cells are stimulated with cytokines and antibodies, and then the CAR gene is introduced using a viral vector or transposon.

In the stable production of T-cell preparations, the selection of a cell population that serves as a starting material is important. In an autologous case, it may be difficult to even secure a sufficient number of cells depending on the patient. Even if a sufficient number of cells can be secured, it may be difficult to produce a stable preparation as a final product depending on the characteristics of the cells.

Naive T cells, collected from a patient's peripheral blood and unstimulated by antigens, are activated and mature into "effector T cells" and "memory T cells". In order to produce a good T cell preparation, it is preferable to maintain the naive-memory state (Non Patent Literature 7).

The properties of T cells contained in T cell sources, including peripheral blood mononuclear cells (PBMCs), influence the phenotype and function of the T cell preparation as a final product.

For example, it has been reported that, in order to improve the production success rate and function of CAR-T cells, activation of T cells and efficiency of CAR gene introduction during the CAR-T cell production process are improved by removing monocytes and granulocytes from the starting materials and concentrating all T cells (Non Patent Literatures 4 to 6). Patent Literatures 1 and 2 disclose a method for activating T cells into which a CAR gene has been introduced and producing CAR-T cells with high antitumor action. Patent Literature 3 discloses a method for producing CAR-T cells by separating a fraction containing T cells by using a specific biomarker by flow cytometry, introducing a gene, and expansion culturing the obtained cells.

Patent Literature 4 discloses a method for producing CAR-T cells by expansion culture including co-culturing T cells, into which genes have been introduced, and cells that have been engineered to express antigens. Patent Literatures 5 and 6 describe a method for activating T cells with a stimulant in the production of CAR-T cells.

However, neither document describes or suggests any method for controlling differentiation of T cells and securing a certain amount of a uniform cell population rich in naive and memory T cells.

### [Citation List]

### [Patent Literature]

[Patent Literature 1]
   WO 2021/020526
[Patent Literature 2]
   WO 2018/110374
[Patent Literature 3]
   US Patent No. 10316289
[Patent Literature 4]
   WO 2021/020526
[Patent Literature 5]
   JP 2021-533746 A
[Patent Literature 6]
   JP 2021-534783 A

### [Non Patent Literature]

[Non Patent Literature 1]
   Rosenberg SA., Nat Rev Clin Oncol. 2011 8(10):577-585.
[Non Patent Literature 2]
   Robbins PF. et al., J Clin Oncol. 2011 29: 917-924.
[Non Patent Literature 3]
   Eshhar Z. et al., Proc Natl Acad Sci U S A, 1993, 90: 720-724.
[Non Patent Literature 4]
   Noaks E. et al., Mol Ther Methods Clin Dev. 2021 20: 675-687.
[Non Patent Literature 5]
   Stroncek DF. et al., Cytotherapy. 2016 18(7): 893-901.
[Non Patent Literature 6]
   Stroncek DF. et al., J Transl Med. 2017 15(1): 59.
[Non Patent Literature 7]
   Arcangeli S. et al., J Clin Invest. 2022 Jun 15; 132(12):e150807.

### [Summary of Invention]

### [Technical Problem]

When performing gene transfer, it is necessary to secure a required number of T cells with certain properties. However, the desired cell population in a necessary quantity may not be secured since the patient's own T cells are used. Therefore, the present invention aims to maintain and proliferate T cells collected from a patient as a cell population suitable for gene transfer.

### [Solution to Problem]

In view of the above-mentioned problem, the present inventors have conducted intensive studies and found that naive and/or memory T cells can be maintained and proliferated by contacting T cells with a certain amount of a stimulant during culture thereof, specifically, by contacting a composition containing T cells with a stimulant that activates (i) one or more intracellular signaling domains of one or more components of a TCR complex, and (ii) one or more intracellular signaling domains of one or more co-stimulatory molecules, and confirmed that the culture method of T cells is useful as a pre-culture method for compositions containing T cells that serve as raw materials for producing T cell preparations, which resulted in the completion of the present invention.

Accordingly, the present invention provides the following.

[1] A method for culturing a T cell, comprising a step of contacting a composition comprising T cells with a stimulant, wherein
   the aforementioned stimulant activates (i) one or more intracellular signaling domains of one or more components of a TCR complex and (ii) one or more intracellular signaling domains of one or more co-stimulatory molecules,
   an amount of the aforementioned stimulant added is 1 to 1500 ng per 10⁶ cells in the composition comprising T cells, and
   a proportion of T cells in the composition comprising T cells is 5% or more of the total number of cells in the composition.
[2] A method for culturing a T cell, comprising a step of contacting a composition comprising T cells with a stimulant, wherein
   the aforementioned stimulant activates (i) one or more intracellular signaling domains of one or more components of a TCR complex and (ii) one or more intracellular signaling domains of one or more co-stimulatory molecules, and
   an amount of the aforementioned stimulant added is 1 to 1500 ng per 10⁶ T cells in the composition comprising T cells.
[3] The method of [1] or [2], comprising a step of separating T cells from the composition comprising T cells.
[4] The method of any of [1] to [3], wherein the aforementioned method for culturing T cells is a method for maintaining or proliferating T cells.
[5] The method of any of [1] to [4], wherein the aforementioned T cell is a naive and/or memory T cell.
[6] The method of any of [1] to [5], wherein the aforementioned stimulant comprises an anti-CD3 antibody or an antigen-binding fragment thereof and an anti-CD28 antibody or an antigen-binding fragment thereof.
[7] The method of [6], wherein a ratio of the anti-CD3 antibody or an antigen-binding fragment thereof to the anti-CD28 antibody or an antigen-binding fragment thereof contained in the aforementioned stimulant is 1:0.5 to 1:200.
[8] The method of any of [1] to [7], wherein the aforementioned stimulant is present on a support.
[9] The method of [8], wherein the aforementioned support is a bead or a nanomatrix.
[10] A method for producing a chimeric antigen receptor T (CAR-T) cell, comprising the following steps:
   (1) a step of culturing T cells by the method of [1] or [2] (pre-culture step),
   (2) a step of introducing the CAR gene into the pre-cultured T cells (CAR-T introduction step), and when desired,
   (3) a step of expansion culturing the cells into which the CAR gene has been introduced (expansion culture step).
[11] An additive for T cell culture, comprising an anti-CD3 antibody or an antigen-binding fragment thereof and an anti-CD28 antibody or an antigen-binding fragment thereof, wherein the T cell is naive and/or memory T cell.
[12] The additive of [11], which is used in a dose of 1 to 1500 ng per 10⁶ T cells.

### [Advantageous Effects of Invention]

According to the method of the present invention, young T cells (e.g., naive and/or memory T cells) can be proliferated while being kept in a young state, whereby a sufficient quantity of T cells suitable for T cell therapy and CAR-T cell therapy can be obtained.

### [Brief Description of Drawings]

[Fig. 1]
A diagram showing the results of flow cytometry of a cell population after 7 days of pre-culture in the presence of stimulants (anti-CD3 antibody and anti-CD28 antibody) (Pre-culture Day 7).

### [Description of Embodiments]

The present invention is described below. Unless otherwise specified, the terms used in the present specification have meanings generally used in the pertinent field.

The present invention provides a culture method of T cells (hereinafter also to be referred to as "the culture method of the present invention"). The method includes a step of contacting a composition containing T cells with a stimulant, and the aforementioned stimulant activates (i) one or more intracellular signaling domains of one or more components of a TCR complex, and (ii) one or more intracellular signaling domains of one or more co-stimulatory molecules.

### composition comprising T cells

The T cells in the "composition comprising T cells" of the present invention (hereinafter also to be referred to as "the T cell-containing composition" of the present invention) may be autologous cells or allogeneic cells. In the present invention, "autologous cell" refers to a cell obtained from a subject who receives administration of a T cell preparation produced using T cells cultured and proliferated by the method of the present invention as raw materials (e.g., activated T cells, CAR-T cells), or a cell induced from the obtained cell, and "allogeneic cell" means that it is not the aforementioned "autologous cell". Preferably, the T cell is an autologous cell. The T cells in the T cell-containing composition include CD4-positive CD8-negative T cells, CD4-negative CD8-positive T cells, CD4-positive CD8-positive T cells, CD4-negative CD8-negative T cells, αβ-T cells, γδ-T cells, Treg cells, NK-like T cells, and NKT cells, and the like. T cells can be subsets such as naive T cells, effector T cells, or memory T cells. T cells may be cells isolated from humans, or cells obtained by differentiation from cells such as iPS cells, ES cells, hematopoietic stem cells, mesenchymal stem cells, and the like. Given that T cell preparations are produced using T cell-containing compositions as raw materials, it is preferable that the T cells are young, unexhausted T cells, such as naive and/or memory T cells.

Unsensitized T cells (naive T cells) proliferate and become activated when they encounter foreign substances such as pathogens, cancer cells, and the like, but eventually most of them die. However, some remain as memory T cells in preparation for the next reinfection or recurrence. Memory T cells are classified into several types with different differentiation stages: stem cell memory T cells (Tscm), central memory T cells (Tcm), and effector memory T cells (Tem). Among these, Tem cells are differentiated and have the strongest attacking power against cancer cells, etc., and Tscm cells are close to naive T cells, are the longest-lived, and are considered to retain immunological memory over a long period. Tscm cells can produce a large number of differentiated Tcm and Tem cells.

In one embodiment, the percentage of naive T cells in the T cell-containing composition is higher than 60%, 65%, 70%, 80%, 90%, or 95% of the total T cells in the T cell-containing composition. In particular embodiments, the naive T cells include CCR7-positive CD45RA-positive (CCR7+CD45RA+), CD27-positive CCR7-positive (CD27+CCR7+), or CD62L-positive CCR7-positive (CD62L+CCR7+) T cells. In some embodiments, the naive T cells include CCR7+CD45RA+ T cells. In particular embodiments, the naive T cells include CD27+CCR7+ T cells.

When T cells are cultured using the method of the present invention, differentiation of T cells do not progress, and naive T cells can proliferate while maintaining their characteristics. Therefore, by culturing the T cell-containing composition using the culture method of the present invention, the percentage of naive T cells in the T cell-containing composition can be increased. Therefore, the percentage of naive T cells in the T cell-containing composition, to which the method of the present invention is applied, may be less than 60%.

In one embodiment, the percentage of memory T cells in the T cell-containing composition is higher than 60%, 65%, 70%, 80%, 90%, or 95% of the total T cells in the T cell-containing composition. In particular embodiments, the memory T cells include CCR7-positive CD45RA-positive (CCR7+CD45RA+), CCR7-positive CD45RA-negative (CCR7+CD45RA-), CCR7-negative CD45RA-negative (CCR7-CD45RA-), or CCR7-negative CD45RA-positive (CCR7-CD45RA+) T cells. In some embodiments, the memory T cells include CCR7+CD45RA+, or CCR7+CD45RA- T cells. In some embodiments, they include CCR7+CD45RA+ T cells.

When T cells are cultured using the method of the present invention, differentiation of T cells do not progress, and memory T cells can proliferate while maintaining their characteristics. Therefore, by culturing the T cell-containing composition using the culture method of the present invention, the percentage of memory T cells in the T cell-containing composition can be increased. Therefore, the percentage of memory T cells in the T cell-containing composition, to which the method of the present invention is applied, may be less than 60%.

In one embodiment, the percentage of naive T cells and/or memory T cells in the T cell-containing composition is higher than 60% of the total T cells in the T cell-containing composition.

The proportion of T cells in the T cell-containing composition is not particularly limited as long as the stimulant of the present invention described later can exert the desired effect, and is preferably 5% or more. In one embodiment, the proportion of T cells in the T cell-containing composition may be 5% or more, 10% or more, 20% or more, 30% or more, 40% or more, 50% or more, 60% or more, 70% or more, 80% or more, 90% or more, 95% or more, or 100%. As described later, if the T cell-containing composition is obtained from or derived from subjects (so-called patients) who have a specific disease or condition, require cell therapy, or undergo cell therapy, the proportion of T cells in the T cell-containing composition may be less than 5%, or about a few percent. In such cases, the T cells in the composition may be concentrated as appropriate, using various methods known per se.

The T cell-containing composition may be the T cell source as is, or obtained by isolating, selecting, or concentrating T cells from the T cell source (processed product). When the T cells are autologous cells, T cell source, for example, a biological sample obtained from or derived subjects who have a specific disease or condition, require cell therapy, or undergo cell therapy is used as the T cell-containing composition. The biological sample is not particularly limited, and includes body fluids, such as blood, plasma, serum, cerebrospinal fluid, synovial fluid, urine, and sweat, tissue and organ samples, and also includes processed samples derived therefrom. Preferably, it is blood or a sample derived from blood, or a product derived from apheresis or leukapheresis or derived therefrom (e.g., whole blood samples, buffy coat samples, peripheral blood mononuclear cell (PBMC) samples, unfractionated T cell samples, lymphocyte samples, leukocyte samples, apheresis products, or leukocyte apheresis products). T cell-containing composition may be cryopreserved.

A preferred T cell-containing composition uses peripheral blood mononuclear cells (PBMCs) or processed products thereof. The PBMC is preferably autologous PBMC. PBMC can be prepared by conventional methods, for example, Saha S, Nakazawa Y, Huye LE, Doherty JE, Galvan DL, Rooney CM, Wilson MH. J Vis Exp. 2012 Nov 5; (69): e4235. Unless particularly indicated, various cells (e.g., T cells) in the present specification are human cells.

A T cell-containing composition with a high proportion of T cells can be obtained by selecting, isolating, or concentrating T cells from a T cell source. For example, selection, isolation, or concentration can be performed by separating and obtaining T cells from a T cell source or separating and removing non-T cells from a T cell source, based on the characteristics of T cells and/or non-T cells. In one example, T cells can be isolated from PBMC samples by negative selection of B cells, monocytes, or other leukocytes using markers expressed on non-T cells (e.g., CD14). In another example, in order to generate a population enriched with CD3-positive (CD3+) T cells from a T cell source such as PBMC or leukocyte apheresis samples, a step of selecting CD3+ T cells can be performed.

The proportions of naive T cells, Tscm, Tcm, and/or Tem in a T cell-containing composition can also be adjusted by positive selection or negative selection based on the surface antigens associated with each subpopulation of T cells.

Specific methods for selecting, isolating, or enriching T cells from a T cell source include the following.

T cells expressing a specific cell membrane surface antigen are separated from cells that do not express the antigen. Such separation step can be performed using a method generally used to separate cells based on the expression status of cell membrane surface antigens. Preferred embodiments of the method of the present invention include density gradient separation method, immunological cell separation technique, magnetic cell separation technique, nylon wool separation method, and adhesion method.

The density gradient separation method (also to be referred to as density gradient centrifugation method) is a technique that allows cells to be separated according to the size, shape, and density thereof. A density gradient can be formed in a container such as a centrifuge tube by layering solutions of various densities that become highly dense at the bottom of a container such as a tube.

The magnetic cell separation technique is also known as a magnetic cell sorting (MACS) method, and involves preparing a cell suspension from tissue containing a mixture of various cells, magnetically labeling specific cells in the suspension, and separating and collecting magnetically labeled cells and non-magnetically labeled cells.

The immunological cell separation technique is a method of separating cells by using antigen-antibody reactions.

The nylon wool separation method is a method that utilizes the property that B cells adhere well to nylon wool.

The adhesion method is a method of separating cells by utilizing differences in the ability of the cells to adhere to a substrate (e.g., culture flask). Specifically, when a T cell source is placed in a culture flask, monocytes etc. that have adhesive ability adhere to the bottom of the culture flask, while lymphocytes such as T cells that do not have adhesive ability float in the culture medium, whereby they are separated into two cell groups using the adhesion method. When cells that adhere to a culture container are removed, a T cell source is left in the culture container for a certain period of time (e.g., 1 second to 7 days), and after lapse of a certain period of time, only the floating cells are collected and used as a T cell source without using the adherent cells. Examples of culture container include, but are not limited to, culture flask, culture chamber, culture bag, culture plate, culture dish, bioreactor, and the like. Furthermore, the material of the culture container is not particularly limited, and polystyrene, TPP, PETG, glass, and the like can be mentioned. The culture container may or may not be coated or surface-treated with a coating agent (e.g., collagen, fibronectin, polylysine, plasma treatment, electric charge treatment, etc.).

These separation methods and separation techniques may be used alone or two or more kinds of separation techniques may be combined. For example, immune density gradient separation method that combines density gradient separation method and immunological cell separation technique, immunomagnetic cell separation technique that combines magnetic cell separation technique and immunological cell separation technique, a technique in which cells are separated by adhering them to a culture container coated with an antibody or the like, by combining adhesion method and immunological cell separation method, and the like can be mentioned. Cell separation using immunological cell separation techniques is performed by labeling cells with antibodies or ligands specific to the surface of the cells to be separated, and performing density gradient fractionation using the label as an indicator. One of the immunomagnetic cell separation techniques is magnetic bead separation using magnetic beads.

### stimulant

In the present invention, a stimulant (hereinafter also referred to as "the stimulant of the present invention") is used by contacting the stimulant with T cells, particularly naive and/or memory T cells, contained in the T cell-containing composition. Here, "contact" includes incubating and/or culturing cells with the stimulant.

By contacting with the stimulant, the survival (maintenance) and proliferation of T cells are promoted. Moreover, the survival (maintenance) and proliferation of T cells in a state susceptible to gene introduction are promoted. Furthermore, when the maintenance and/or proliferation of young T cells such as naive T cells, memory T cells, and the like are/is supported, and CAR-T cells are prepared from such young T cells, a high antitumor effect can be imparted to CAR-T cells. Preferred memory T cells include stem cell memory T cells (Tscm).

In the present invention, the stimulant activates (i) one or more intracellular signaling domains of one or more components of a TCR complex, and (ii) one or more intracellular signaling domains of one or more co-stimulatory molecules.

In one embodiment, the stimulant of the present invention contains a combination of one or more active agents (active agent 1) capable of activating the intracellular signaling domain of a TCR complex and one or more active agents (active agent 2) capable of activating one or more intracellular signaling domains of one or more co-stimulatory molecules. Active agent 1 and active agent 2 may be the same or different, and are preferably different. Where "active agent" is denoted in the present specification, it includes both active agent 1 and active agent 2. Active agents include, for example, ligands. Active agents envisioned in the present specification may include, but are not limited to, RNA, DNA, proteins (e.g., enzymes), antigens, polyclonal antibodies, monoclonal antibodies, antibody fragments, carbohydrates, lipid lectins, and any other biomolecules having affinity for a desired target. In some embodiments, the desired target is a T cell receptor and/or a component of a T cell receptor. In a particular embodiment, the desired target is CD3. In a particular embodiment, the desired target is a T cell co-stimulatory molecule, such as CD28, 4-1BB (CD137), OX40, or ICOS. A T cell co-stimulatory molecule is a molecule that activates T cells through cooperative action (secondary signal) with TCR (primary signal).

In some embodiments, the stimulant contains one or more active agents (e.g., an antibody or an antigen-binding fragment thereof) attached to a support and specifically binding to one or more of the following macromolecules on a cell (e.g., a T cell), namely CD2, CD3, CD4, CD5, CD8, CD25, CD27, CD28, CD29, CD31, CD44, CD45RA, CD45RO, CD54 (ICAM-1), CD127, MHCI, MHCII, CTLA-4, ICOS, PD-1, OX40, CD27L (CD70), 4-1BB (CD137), 4-1BBL, CD30L, LIGHT, IL-2R, IL-12R, IL-1R, IL-15R, IFN-γR, TNF-αR, IL-4R, IL-10R, CD18/CD1la (LFA-1), CD62L (L-selectin), CD29/CD49d (VLA-4), Notch ligands (e.g., Delta-like 1/4, Jagged 1/2, etc.), CCR1, CCR2, CCR3, CCR4, CCR5, CCR7, and CXCR3, or fragments thereof including corresponding ligands for these macromolecules or fragments thereof.

In some embodiments, the active agent (e.g., antibody) attached to a support specifically binds to one or more of the following molecules on T cells: CD28, CD62L, CCR7, CD27, CD127, CD3, CD4, CD8, CD45RA, and/or CD45RO.

Therefore, in one preferred embodiment of the present invention, what activates (i) one or more intracellular signaling domains of one or more components of a TCR complex, and (ii) one or more intracellular signaling domains of one or more co-stimulatory molecules is a combination of (i) one that activates one or more intracellular signaling domains of one or more components of a TCR complex (active agent 1), and (ii) one that activates one or more intracellular signaling domains of one or more co-stimulatory molecules (active agent 2).

The ratio of active agent 1 to active agent 2 is not particularly limited as long as the desired effect is obtained, and active agent 1:active agent 2 is generally 1:0.5 to 200, preferably 1:0.5 to 100, more preferably 1:0.5 to 50, further preferably 1:0.5 to 10, particularly preferably 1:0.5 to 2, most preferably 1:1.

In some embodiments, one or more active agents are antibodies. Antibodies may include polyclonal antibodies, monoclonal antibodies (including full-length antibodies having an immunoglobulin Fc region), antibody compositions having polyepitope specificity, multispecific antibodies (e.g., bispecific antibodies, diabodies, and single-chain molecules), and antibody fragments (e.g., Fab, F(ab')₂, and Fv). In some embodiments, the active agent is an antibody fragment (including an antigen-binding fragment), for example, Fab, Fab'-SH, Fv, scFv, or F(ab')₂ fragment. Constant region of any isotype can be used in the antibodies assumed in the present specification, including IgG, IgM, IgA, IgD, and IgE constant regions, and it is recognized that such constant regions can be obtained from any human or animal species (e.g., mouse species). In some embodiments, the active agent is an antibody that binds to and/or recognizes one or more components of a T cell receptor.

As active agent 1, an anti-CD3 antibody or an antigen-binding fragment thereof is preferred, and as active agent 2, an anti-CD28 antibody or an antigen-binding fragment thereof is preferred. An anti-CD3 antibody is particularly preferred as active agent 1, and an anti-CD28 antibody is particularly preferred as active agent 2.

In some embodiments, the contact conditions between the T cell-containing composition and the stimulant are appropriately set according to the type of stimulant used and the proportion of cells, particularly T cells, in the T cell-containing composition. Generally, 1 to 1500 ng, preferably 1 to 1000 ng, preferably 1 to 500 ng, preferably 1 to 200 ng, preferably 1 to 150 ng, preferably 1 to 100 ng, more preferably 1 to 50 ng, further preferably 1 to 20 ng, particularly preferably 1 to 10 ng, are used per 10⁶ cells in the T cell-containing composition. In some embodiments, the contact conditions between the T cell-containing composition and the stimulant are appropriately set according to the type of stimulant used, and generally, 1 to 1500 ng, preferably 1 to 1000 ng, preferably 1 to 500 ng, preferably 1 to 200 ng, preferably 1 to 150 ng, preferably 1 to 100 ng, more preferably 1 to 50 ng, particularly preferably 1 to 10 ng, are used per 10⁶ T cells in the T cell-containing composition.

Although not shown in the aforementioned examples, embodiments in which a stimulant is added to a medium and simultaneously bound to a support, and the like may also be employed.

Examples of supports to which the stimulant is bound include film-like, sponge-like, fibrous, rod-like, bead-like, and colloidal (e.g., gel-like) supports, and a bead-like (particle size 10 nm to 500 µm, preferably 10 nm to 100 µm, more preferably 10 nm to 50 µm) and a gel-like support are particularly preferred. Furthermore, a nanomatrix structure is also a preferred embodiment. As the material types, magnetic substance, latex, agarose, glass, cellulose, sepharose, nitrocellulose, polystyrene, retronectin, collagen, and the like can be mentioned.

In one embodiment, the stimulant contains particles (e.g., beads) conjugated or linked to one or more active agents (e.g., biomolecules) capable of expanding and proliferating T cells. In some embodiments, one or more active agents are bound to the beads. In some embodiments, the beads are biocompatible, i.e., made of a material suitable for biological use. In some embodiments, the beads are non-toxic to cultured T cells.

One or more active agents can also be used while being directly or indirectly attached to a support by a wide variety of methods known and available in the art. Attachment can be covalent, non-covalent, electrostatic, or hydrophobic, and can be achieved by a wide variety of attachment means, including, for example, chemical means, mechanical means, or enzymatic means. In some embodiments, the active agent is an antibody or an antigen-binding fragment thereof (e.g., Fab). In some embodiments, a biomolecule (e.g., a biotinylated anti-CD3 antibody) may be indirectly attached to the beads via another biomolecule (e.g., an anti-biotin antibody) directly attached to the support.

In one embodiment of the present invention, the stimulant is present on the surface of the beads as a support, or attached to them and used. In particular embodiments, the beads have diameters larger than 1 µm and 9 µm or less, 8 µm or less, 7 µm or less, 6 µm or less, or 5 µm or less. The bead:cell ratio is 100:1 to 1:100, preferably 60:1 to 1:60, more preferably 20:1 to 1:60, further preferably 10:1 to 1:60.

In one embodiment, the cells in the T cell-containing composition are cultured at a density of 0.1, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10, 20, 30, 40, or 50×10⁶ cells/mL, preferably 1.0, 1.5, 2.0, 2.5, 3.0, 3.5, 4.0, 4.5, 5.0, 5.5, 6.0, 6.5, 7.0, 7.5, 8.0, 8.5, 9.0, 9.5 or 10×10⁶ cells/mL, more preferably 5.0, 5.5, 6.0, 6.5, 7.0, 7.5 or 8.0×10⁶ cells/mL, in the presence of a stimulant. In a particular embodiment, T cells, for example, T cells in a T cell-containing composition, are cultured at a density of 0.1 to 50×10⁶ cells/mL, preferably 1.0 to 10×10⁶ cells/mL, more preferably 5.0 to 8.0×10⁶ cells/mL, in the presence of a stimulant. The cell density can be appropriately set according to the proportion of T cells in the T cell-containing composition.

The medium used during culture of T cells in the present invention is not particularly limited as long as it can be used in the presence of a stimulant, and a medium used for normal cell culture, such as RPMI1640, MEM, X-VIVIO, IMDM, DMEM, DC medium, OptiMEM, and the like can be used. The medium may be a medium to which serum (human serum, fetal bovine serum, etc.) is added according to a conventional method, or a serum-free medium. It is preferable to use a serum-free medium because it is highly safe for clinical application and the culture efficiency is less likely to vary due to differences between serum lots. Examples of serum-free media include TexMACS TM (Miltenyi Biotec), AIM V (registered trademark) (Thermo Fisher Scientific), ALyS culture medium (Cell Science & Technology Institute, Inc.), and the like. When using serum, autologous serum, that is, serum collected from the individual from whom the CAR-expressing immune cells are derived (more specifically, a patient receiving administration of the cell population obtained by the production method of the present disclosure) may also be used, or it may also be an artificial serum. In the present invention, it is preferable to use artificial serum. Plasma may also be used. Blood components such as serum or plasma, albumin, etc. or analogs thereof may be used, and artificial substances may also be used. As the basal medium, one suitable for cell culture may be used, and the aforementioned TexMACS TM (Miltenyi Biotec), AIM V (registered trademark), and ALyS culture medium (Cell Science & Technology Institute, Inc.) can be used. Other culture conditions may be those suitable for cell survival and proliferation, and general conditions may be employed. For example, culturing in a CO₂ incubator (CO₂ concentration 5%) set at 37°C, hypoxic culture (oxygen concentration 0 to 20%, preferably 0 to 10%, more preferably 1 to 5%), and the like can be mentioned. The culture time is the time until the desired amount of T cells is obtained, and varies depending on the seeding concentration. It is generally about 2 to 10 days, 3 to 9 days, 4 to 8 days, or 5 to 7 days, and the culture medium is appropriately added or replaced as necessary.

Additional factors may be added to the medium in order to support cell survival and proliferation. As the additional factor, type 1 cytokine family member, type 2 cytokine family member, TNF superfamily cytokine, IL-1 family cytokine, other cytokines (TNF-β, etc.) can be mentioned, and specifically IL-1 to IL-41 and the like, preferably IL-1, IL-2, IL-7, IL-15, IL-21, and the like, can be mentioned. When preparing CAR-T cells (described later), IL-7 and/or IL-15 may be added to the medium. Additional factors can be prepared according to conventional methods, and commercially available products can also be used. Additional factors may be from an animal species other than humans, but are preferably derived from humans (and may be recombinant).

The thus-obtained T cell-containing composition, expansion cultured in the presence of a stimulant, may have a proportion of naive or young memory T cells of 20%, 30%, 45%, 50%, 55%, or 60% or more, preferably 60% or more. Therefore, the T cell-containing composition expansion cultured in the presence of a stimulant can be and is preferably used as a starting material for T cell immunization or the production of CAR-T cells. As one example, the production of CAR-T cells using a T cell-containing composition obtained by the culture method of the present invention is described.

### production of CAR-T cells

### CAR

CAR is a structure that contains, from the N-terminal side to the C-terminal side of a protein, a target-specific extracellular domain, a transmembrane domain, and an intracellular signal domain for effector functions of immune cells, and CAR gene is a gene encoding this receptor. The extracellular domain contains an antigen recognition site that exhibits target-specific binding. The transmembrane domain exists between the extracellular domain and the intracellular signal domain. The intracellular signal domain transmits signals necessary for immune cells to exert their effector functions. That is, when the extracellular domain binds to a target antigen, an intracellular signal domain is used that is capable of transmitting a signal necessary for activating immune cells.

There have been several reports of experiments and clinical studies using CAR, and the CAR can be constructed by referring to these reports (e.g., Rossig C, et al. Mol Ther 10:5-18, 2004; Dotti G, et al. Hum Gene Ther 20:1229-1239, 2009; Ngo MC, et al. Hum Mol Genet 20 (R1):R93-99, 2011; Ahmed N, et al. Mol Ther 17:1779-1787, 2009; Pule MA, et al. Nat Med 14:1264-1270, 2008; Louis CU, et al. Blood 118:6050-6056, 2011; Kochenderfer JN, et al. Blood 116:4099-4102, 2010; Kochenderfer JN, et al. Blood 119:2709-2720, 2012; Porter DL, et al. N Engl J Med 365:725-733, 2011; Kalos M, et al. Sci Transl Med 3:95ra73,2011; Brentjens RJ, et al. Blood 118:4817-4828, 2011; Brentjens RJ, et al. Sci Transl Med 5:177 ra38, 2013).

### introduction of CAR gene into T cells

CAR-T cells are prepared by introducing a CAR gene into T cells by a CAR expression vector. The T cells into which the CAR gene is introduced are preferably T cells in a T cell-containing composition expansion cultured by the above-mentioned culture method of the present invention. The CAR expression vector means a molecule capable of transporting a nucleic acid molecule encoding a CAR gene into T cells. It is not questioned whether nucleic acid molecule is DNA or RNA and is not particularly limited as to the form, origin, and the like, and various kinds of vectors can be used. The vector may be a viral vector or a non-viral vector. As the viral vector, retroviral vector, lentiviral vector, adenoviral vector, adeno-associated viral vector, herpes virus vector, sendai virus vector, vaccinia virus vector, poxvirus vector, phage, and the like can be mentioned. Among these, with retroviral vectors, lentiviral vectors, and adeno-associated viral vectors, the target gene incorporated into the vector is integrated into the host chromosome, and stable and long-term expression can be expected. Each viral vector can be produced according to a conventional method or using a commercially available dedicated kit. Non-viral vectors include plasmid vectors, liposome vectors, positively charged liposome vectors (Felgner, P.L., Gadek, T.R., Holm, M. et al., Proc. Natl. Acad. Sci., 84:7413-7417, 1987), YAC vectors, BAC vectors, artificial chromosome vectors, cosmid vectors, and the like.

A CAR expression vector contains an expression unit for expressing a CAR gene, and the expression unit generally contains a promoter, a CAR gene, and a poly A addition signal. The expression unit is derived from various organisms or viruses or is composed of any sequence, and includes similar sequences and modified units thereof. Promoters that can be used in the CAR expression cassette include CAG promoter, CMV-IE (cytomegalovirus early gene-derived promoter), SV40ori, retrovirus LTRSRα, EF1α, β actin promoter, and the like. Examples of the poly A addition signal sequence include poly A addition sequence of SV40, poly A addition sequence of bovine-derived growth hormone gene, globulin poly A addition sequence, and the like. In order for the expression of the CAR gene to be controlled by the promoter, the CAR gene is generally linked directly to the 3'-terminal side of the promoter or via other sequence, and a poly A addition signal sequence is configured downstream of the CAR gene. The CAR gene is transcribed into messenger RNA (mRNA) by such expression unit, and CAR is translated from the mRNA and displayed on the cell surface.

The expression unit may contain gene for detection (reporter gene, cell- or tissue-specific gene, selection marker gene, and the like) to enable detection of gene expression, an enhancer sequence to improve expression efficiency, a WRPE sequence, and the like.

The gene for detection is used for determining the success or failure and efficiency of introduction of the CAR expression vector; detecting the expression or determining the expression efficiency of the CAR gene; and selecting and sorting cells in which the CAR gene is expressed, and the like. As the gene for detection, genes such as the neo gene that confers resistance to neomycin, kmr gene and nptII gene that confer resistance to kanamycin and the like (Bernd Reiss et al. EMBO J. 3 (1984), 3317-3322), hph gene that confers resistance to hygromycin (Blochlinger & Diggelmann, Mol Cell Bio 4:2929-2931), DHFR gene that confers resistance to methotrexate (Bourouis et al., EMBO J.2(7)), and the like (all marker genes); luciferase gene (Giacomin, P1. Sci. 116(1996), 59-72; Scikantha, J. Bact. 178(1996), 121), β-glucuronidase (GUS) gene, genes for fluorescent proteins such as GFP (Gerdes, FEBS Lett. 389(1996), 44-47) or variants thereof (EGFP, d2EGFP, and the like), and the like (all reporter genes); epidermal growth factor receptor (EGFR) gene, which lacks an intracellular domain, and the like can be used. The gene for detection may be linked to the CAR gene via, for example, a bicistronic control sequence (e.g., internal ribosome entry site (IRES)) or a sequence encoding a self-cleavage peptide. As the self-cleavage peptide, 2A peptide (T2A) derived from Thosea asigna virus can be mentioned. Examples of other self-cleavage peptides include, but are not limited to, 2A peptide derived from picornavirus, 2A peptide (F2A) derived from foot-and-mouth disease virus (FMDV), 2A peptide (E2A) derived from equine rhinitis A virus (ERAV), 2A peptide (P2A) derived from porcine teschovirus (PTV-1), and the like, 2A peptide derived from rotavirus, insect virus, aphthovirus, or trypanosoma virus, and the like. Similar sequences and partially modified sequences can also be mentioned.

The CAR gene expression vector prepared for gene transfer is introduced into T cells by a conventional method. In the case of viral vectors, they are introduced into cells by infection with the virus. In the case of non-viral vectors such as plasmids, conventional methods such as electroporation method, liposomes method, calcium phosphate method, nucleofection method, laser method, cation method, microinjection method, sonoporation, and the like are used for introduction into cells, and electroporation method is preferably used for introduction.

In order to improve the efficiency of integration into the host chromosome, it is preferable to perform gene introduction by the transposon method. The transposon method is one of nonviral gene introduction methods that uses a pair of gene enzyme (transposase) and its specific recognition sequence to cause gene transposition, and can integrate any gene into the host chromosome. As the transposon method, for example, the piggyBac transposon method can be used. The piggyBac transposon method utilizes transposons isolated from insects (Fraser MJ et al., Insect Mol Biol. 1996 May; 5(2):141-51.; Wilson MH et al., Mol THER2007 Jan; 15(1):139-45.), and enables highly efficient integration into mammalian chromosomes. The piggyBac transposon method is actually used for gene introduction (e.g., Nakazawa Y, et al., J Immunother 32:826-836, 2009; Nakazawa Y et al., J Immunother 6:3-10, 2013, and the like).

The transposon method is not limited to one using piggyBac and may also be, for example, a method using transposon, such as Sleeping Beauty (Ivics Z, Hackett PB, Plasterk RH, Izsvak Z (1997) Cell 91: 501-510.), Frog Prince (Miskey C, Izsvak Z, Plasterk RH, Ivics Z (2003) Nucleic Acids Res 31: 6873-6881.), Tol1 (Koga A, Inagaki H, Bessho Y, Hori H. Mol Gen Genet. 1995 Dec 10; 249(4):400-5.; Koga A, Shimada A, Kuroki T, Hori H, Kusumi J, Kyono-Hamaguchi Y, Hamaguchi S. J Hum Genet. 2007; 52(7):628-35. Epub 2007 Jun 7.), Tol2 (Koga A, Hori H, Sakaizumi M (2002) Mar Biotechnol 4: 6-11.; Johnson Hamlet MR, Yergeau DA, Kuliyev E, Takeda M, Taira M, Kawakami K, Mead PE (2006) Genesis 44: 438-445Choo BG, Kondrichin I, Parinov S, Emelyanov A, Go W, Toh WC, Korzh V (2006) BMC Dev Biol 6: 5.), and the like.

The operation of gene introduction using the transposon method can be performed by a conventional method. For example, for the piggyBac transposon method, a vector carrying a gene encoding piggyBac transposase (transposase plasmid) and a vector having a structure in which the CAR gene expression unit is sandwiched between piggyBac inverted repeat sequences (transposon plasmid) are prepared, and these vectors can be introduced into target cells by various techniques such as electroporation, nucleofection, lipofection, and calcium phosphate method.

### antigen recognition treatment and expansion culture of CAR-T cells after CAR gene introduction

As a treatment after CAR gene introduction, an antigen recognition treatment can be further performed to impart CAR-T cells with antitumor property. The method of the antigen recognition treatment is not particularly limited as long as the desired effect on CAR-T cells can be obtained. For example, it can be performed by contacting with a recognition substance which is an antigen to be the target of CAR. By performing an antigen recognition treatment, CAR-T cells can specifically recognize antigens. Furthermore, they become CAR-T cells that have antitumor property against the recognized antigen.

As the recognition substance, a tumor-associated antigen or tumor specific antigen that is targeted by CAR, for example, EPHA2, HER2, EPHB2, EPHB4, EGFR, GD2, Glypican-3, HER2, 5T4, 8H9, αvβ6 integrin, B cell maturation antigen (BCMA), B7-H3, B7-H6, CAIX, CA9, CD19, CD20, CD22, κ light chain, CD30, CD33, CD38, CD44, CD44v6, CD44v7/8, CD70, CD116, CD123, CD138, CD171, CEA, CSPG4, EGFR, EGFRvIII, EGP2, EGP40, EPCAM, ERBB3, ERBB4, ErbB3/4, FAP, FAR, FBP, fetal AchR, folate receptor α, GD3, HLA-AI MAGE A1, HLA-A2, IL11Ra, IL13Ra2, KDR, lambda, Lewis Y, MCSP, mesothelin, MUC1, MUC4, MUC6, NCAM, NKG2D ligand, NY-ESO-1, PRAME, PSCA, PSC1, PSMA, ROR1, Sp17, SURVIVIN, TAG72, TEM1, TEM8, VEGR receptor 2, oncofetal antigen, HMW-MAA, VEGF receptor, fibronectin, tenascin, factors that enhance cancer migration (e.g., chemokines) or antigens present in the extracellular matrix, such as carcinoembryonic antigen (CEA) in necrotic areas of the tumor, or proteins containing mutations identified by genomic analysis and/or differential expression study of tumor, and the like can be mentioned. EPHB4, GD2, HER2, and CD19 are preferred, and EPHB4, HER2, and CD19 are more preferred.

Contact between the recognition substance, which is the antigen targeted by CAR, and CAR-T cells may, for example, be contact between the CAR-T cells and a support to which the recognition substance is bound.

Examples of the support to which a recognition substance is bound include respective supports described in the above-mentioned "stimulant" section. Preferably, the support is bead-shaped (particle size of 10 nm to 500 µm, preferably 10 nm to 100 µm, more preferably 10 nm to 50 µm) or gel-shaped.

A co-stimulatory factor may also be bound to the support in addition to the recognition substance, and a co-stimulatory factor is bound in a preferred embodiment. Examples of co-stimulatory factors include CD40, CD80, 4-1BBL, OX40, OX40L, CD52, CD54, CD70, CD58, CD86, CD95, CD252, CD275, and the like, and ligands of the integrin family (CD49a to CD49h, CD51, CD103, CD41, CD11a to 11c, ITGA9 to 11, CD18, CD19, CD61, ITGB4 to 8, etc.). In one embodiment, the co-stimulatory factor is one or more co-stimulatory factors selected from CD40, CD80, 4-1BBL, and OX40L, preferably CD80 and/or 4-1BBL, more preferably CD80 and 4-1BBL.

In one preferred embodiment, beads conjugated with EPHB4-CD80-4-1BBL are used for antigen recognition treatment of CAR-T cells after CAR gene introduction.

In addition, the tumor-associated antigen or tumor-specific antigen that is targeted by CAR can be used as cells expressing them. That is, CAR-T cells having antitumor property can be expansion cultured by co-culturing them as feeder cells.

This feeder cell is a cell that has been engineered to express part or all of the target antigen on the cell surface so that the CAR introduced into the CAR-T cell can bind to the target antigen. The target antigen includes, for example, tumor-associated antigens or tumor-specific antigens that are targets of the aforementioned CAR. Feeder cells can be prepared by, as an operation to express a target antigen and as described above for CAR-T cells, introducing a gene encoding the target antigen into cells by using a vector having an expression unit for expressing the target antigen gene. Alternatively, feeder cells can also be prepared by producing mRNA of a target antigen gene and directly introducing the mRNA into cells. In some embodiment, the target antigen and a co-stimulatory factor may be expressed on the surface of target antigen expressing cells by an operation to introduce genes for co-stimulatory factor into cells together with a tumor-associated antigen gene or tumor specific antigen gene.

The obtained CAR-T cells can be expansion cultured ex vivo until a sufficient quantity is obtained.

The medium used for preparation and expansion culture of CAR-T cells is not particularly limited, and a medium used for normal cell culture, such as RPMI1640, MEM, X-VIVIO, IMDM, DMEM, DC medium, OptiMEM, and the like can be used. The medium may be a medium to which serum (human serum, fetal bovine serum, etc.) is added according to a conventional method, or a serum-free medium. It is preferable to use a serum-free medium because it is highly safe for clinical application and the culture efficiency is less likely to vary due to differences between serum lots. Examples of serum-free media include TexMACS TM (Miltenyi Biotec), AIM V (registered trademark) (Thermo Fisher Scientific), ALyS culture medium (Cell Science & Technology Institute, Inc.), and the like. When using serum, autologous serum, that is, serum collected from the individual from whom the CAR-expressing immune cells are derived (more specifically, a patient receiving administration of the cell population obtained by the production method of the present disclosure) may also be used, or it may also be an artificial serum. In the present invention, it is preferable to use artificial serum. Plasma may also be used. Blood components such as serum or plasma, albumin, etc. or analogs thereof may be used, and artificial substances may also be used. As the basal medium, one suitable for cell culture may be used, and the aforementioned TexMACS TM (Miltenyi Biotec), AIM V (registered trademark), and ALyS culture medium (Cell Science & Technology Institute, Inc.) can be used. Other culture conditions may be those suitable for cell survival and proliferation, and general conditions may be employed. For example, culturing in a CO₂ incubator (CO₂ concentration 5%) set at 37°C, hypoxic culture (oxygen concentration 0 to 20%, preferably 0 to 10%, more preferably 1 to 5%), and the like can be mentioned.

Additional factors may be added to the medium in order to support cell survival and proliferation. As the additional factor, type 1 cytokine family member, type 2 cytokine family member, TNF superfamily cytokine, IL-1 family cytokine, other cytokines (TNF-β, etc.) can be mentioned, and specifically IL-1 to IL-41 and the like, preferably IL-1, IL-2, IL-7, IL-15, IL-21, and the like, can be mentioned. When preparing CAR-T cells, IL-7 and/or IL-15 may be added to the medium. Additional factors can be prepared according to conventional methods, and commercially available products can also be used. Additional factors may be from an animal species other than humans, but are preferably derived from humans (and may be recombinant).

In some embodiments, T cells can be cultured in the presence of a T cell activating ligand, which is preferable. The T cell activating ligand used in the present invention is not particularly limited as long as it is a molecule that interacts with T cell surface molecules to promote T cell activation and/or proliferation. Examples include molecules that specifically bind to CD3, which conjugates to TCR and mediates TCR-mediated signal transduction, and surface molecules known as T cell activation co-stimulatory factors such as CD28, CD80/CD86, ICOS, 4-1BB (CD137), OX40, CD27, GITR, BAFFR, TACI, BMCA, CD40L, and the like, and have the function of transmitting activation/proliferation signals or co-signals into T cells or antigen-presenting cells. Such molecules may be physiological ligands (or receptors) for the above-mentioned T cell surface molecules, or non-physiological ligands (or receptors) having agonist activity. As a non-physiological ligand, agonist antibodies can be preferably mentioned.

Using a T cell-containing composition expansion cultured by the T cell culture method of the present invention, a CAR-T cell population that is expected to be highly effective compared to conventional methods can be efficiently produced. For example, the CAR-T cell population obtained using T cell-containing composition expansion cultured by the T cell culture method of the present invention may have a proportion of CAR-T cells of 20%, 30%, or 40% or more, preferably 40% or more.

When T cells continue to be chronically activated, they highly express a large number of immune checkpoint molecules, and become unable to proliferate or attack target cells. This phenomenon is called exhaustion, and exhausted T cells have weak ability to proliferate and attack cancer even when returned to the body, and cannot be expected to show a high therapeutic effect. In the T cells in the T cell-containing composition obtained by the T cell culture method of the present invention, exhaustion is suppressed. The degree of exhaustion can be evaluated by methods practiced in the art. For example, it can be evaluated by examining the expression status of exhaustion markers, which is also convenient. Exhaustion markers include Programmed death 1 (PD-1), T-cell immunoglobulin mucin-3 (Tim-3), lymphocyte activation gene 3 (LAG3), Adenosine A2a receptor (A2aR), Cytotoxic T-lymphocyte-associated antigen 4 (CTLA4), T cell immuno-receptor with Ig and ITIM domains (TIGIT), and the like, and particularly PD-1 can be mentioned. Expression of exhaustion markers can be detected using antibodies against these markers. The CAR-T cell obtained by the T cell-containing composition expansion cultured by the T cell culture method of the present invention has low expression of the exhaustion marker PD-1. For example, the proportion of PD-1 expressing cells among CAR-T cells is less than 10%, preferably less than 5%, more preferably less than 1%.

A cell population containing the thus-obtained CAR-T cells can be used for the treatment of cancer, particularly for the treatment of cancer that expresses the target antigen of the CAR-expressing immune cells. The cancer may be a solid tumor or a hematologic tumor. Specific examples of the cancer include, but are not limited to, various B-cell lymphoma (follicular malignant lymphoma, diffuse large B-cell malignant lymphoma, mantle cell lymphoma, MALT lymphoma, intravascular B-cell lymphoma, CD20-positive Hodgkin lymphoma, and the like), myeloproliferative neoplasm, myelodysplastic/myeloproliferative neoplasm (CMML, JMML, CML, MDS/MPN-UC), myelodysplastic syndrome, acute myeloid leukemia, neuroblastoma, brain tumor, Ewing's sarcoma, osteosarcoma, retinoblastoma, small cell lung cancer, non-small cell lung cancer, melanoma, bone and soft tissue sarcoma, kidney cancer, pancreatic cancer, malignant mesothelioma, prostate cancer, breast cancer, endometrial cancer, cervical cancer, ovarian cancer, colorectal cancer, and the like. In preferred embodiments, the cancer is a solid tumor. Examples of the solid tumor include neuroblastoma, brain tumor, Ewing's sarcoma, osteosarcoma, retinoblastoma, small cell lung cancer, non-small cell lung cancer, melanoma, ovarian cancer, rhabdomyosarcoma, bone and soft tissue sarcoma, kidney cancer, pancreatic cancer, malignant mesothelioma, prostate cancer, breast cancer, endometrial cancer, cervical cancer, ovarian cancer, colorectal cancer, and the like.

A cell population containing CAR-T cells is administered in a therapeutically effective amount that is appropriately determined according to the age, weight, body surface area, symptoms, and the like of the subject. The subject in the present disclosure is generally a human, preferably a cancer patient. A cell population containing CAR-T cells produced by the method of the present invention can be administered at, for example, 1x10⁴ cells to 1x10¹⁰ cells per dose. The route of administration is not particularly limited, and the cells can be administered intratumorally, peritumorally, intraventricularly, intravenously, intraarterially, intraportally, intradermally, subcutaneously, intramuscularly, or intraperitoneally. The cell population of the present disclosure may be administered systemically or locally, and local administration includes direct injection into the target tissue, internal organ, or organ. The administration schedule is appropriately determined according to the age, body weight, body surface area, symptom, and the like of the subject, and may be a single administration or continuous or periodic multiple administrations.

A composition containing a cell population containing CAR-T cells may contain, in addition to the cell population to be administered to a subject, components such as dimethyl sulfoxide (DMSO), serum albumin, and the like for the purpose of cell protection, antibiotics and the like for the purpose of preventing bacterial contamination, various components (vitamins, cytokine, mineral, carbon source, nitrogen source, trace metal, electrolyte, growth factor, steroid, etc.) for the purpose of cell activation, proliferation, or differentiation induction, and the like. The composition can be prepared by conventional methods.

The present invention is described in detail below using examples, but the present invention is not limited in any way. Reagents and materials to be used are commercially available unless otherwise specified, or can be prepared from known literature and the like. In addition, those skilled in the art understand that other substances having similar effects and actions can be used alternatively.

### [Example]

### Example 1: culture of T cells and production of CAR-T cells

CAR-T cells were produced after pre-culturing T cells, using cryopreserved peripheral blood mononuclear cells (PBMCs). (material and method)

### 1. cell preparation

7.5 µg each of anti-CD3 antibody and anti-CD28 antibody (BioLegend) were added to 1×10⁸ maxi beads (Miltenyi Biotec) and conjugated at 4°C for 2 hr (antibody-conjugated beads).

PBMC (HEM) was thawed in a 37°C water bath, and the cells were resuspended in complete medium containing 5% artificial serum (Animal-free; Cell Science & Technology Institute), IL-7 (10 ng/mL; Miltenyi Biotec), and IL-15 (5 ng/mL; Miltenyi Biotec) in ALySTM705 Medium (Cell Science & Technology Institute). The cells were then seeded at 7.5×10⁶ cells/1 mL/well on a 48-well plate (Corning).

10 ng of the previously prepared antibody-conjugated beads, diluted in complete medium, was added, and the cells were cultured by incubation at 37°C in 5% CO₂ for 7 days. The antibody-conjugated beads were used such that anti-CD3 antibody and anti-CD28 antibody were each 10 ng.

Culturing was performed by adding complete medium every 2-3 days and expanding onto a 24-well plate or T25 flask (Corning).

Cell count measurement and FCM analysis were performed on Day 7 (pre-culture Day 7).

### 2. gene transfer

The cell suspension after pre-culture was centrifuged at 300 × g for 5 min at room temperature, and the obtained cell pellet was suspended in electroporation buffer (Miltenyi Biotec) at 300 µL/sample at room temperature. Plasmid DNA (5 µg/µL in physiological saline) was added to the cell suspension as follows.
- PB transposase plasmid: 4 µL per 300 µL of electroporation buffer
- pIRII-EPHB4-28z plasmid: 4 µL per 300 µL of electroporation buffer

The suspension of cell and DNA was placed in an electroporation cuvette (Mirus), and each plasmid DNA was introduced into about 7.5×10⁶ PBMCs by electroporation using a Miltenyi Biotec Prodigy electroporator unit.

### 3. expansion culture

After gene transfer, the cells were transferred to complete medium and cultured for about 14 days (CAR-T cells Day 14). On day 1 and day 8 after electroporation, 3×10⁶ beads conjugated with EPHB4-CD80-4-1BBL were added, and expansion culture was performed.

As the culture vessel, the cells were seeded in 48-well plates (Corning), and then scaled up as the cells proliferated. On Day 14, cell count measurement, FCM analysis, and anti-cancer assay were performed.

### (analysis and results)

### 1. cell count measurement

CAR-T cell count was measured using NC-3000 (MS Techno Systems). The program Viability and Cell Count Assay, which can analyze live cell count, dead cell count, viability, cell diameter, and aggregation rate, was used.

### 2. flow cytometry (FCM) measurement

Cells were stained using anti-Ephrin B2 antibody (R&D), anti-CD3 antibody, anti-CD45RA antibody, anti-CCR7 antibody, and anti-PD-1 antibody (BioLegend), and the analysis was performed using MACSQuant (registered trademark) analyzer 10 (Miltenyi Biotec).

Antibody staining was performed by adding the manufacturer's specified per-test amount to 1×10⁶ cells/100 µL of D-PBS at 4°C, and reacting for 15 min at 4°C. Primary staining involved the addition of anti-Ephrin-B2 antibody, secondary staining involved the addition of the secondary antibody of the primary stain, and tertiary staining involved the simultaneous addition of all remaining antibodies. A washing step with 1 mL of D-PBS was included between each staining.

CAR positivity and PD-1 levels were measured by staining dead cells with 7-AAD (Miltenyi Biotec). 7-AAD negative cell group and CD3 positive cell group were analyzed using FCM. MACSQuantify^{™} analyzer software was used for analysis of the results.

### 3. anti-cancer assay

The assay was performed using an in vitro method. The cancer cells used were Rh30 cells (ATCC), a human rhabdomyosarcoma cell line.

For each condition, cancer cells were prepared in a medium to 2×10⁵ cells/1 mL/well, seeded on a 24 well cell bind plate (Corning), and cultured in an adherent culture at 37°C in a 5% CO₂ incubator for 2 to 24 hr.

Thereafter, CAR-T cells were seeded at CAR-T cells:cancer cells=4:1 and cultured at 37°C in a 5% CO₂ incubator for 3 days (co-culture Day 3). The medium used during co-culture was Rh30 cell medium (RPMI1640-10% FBS medium), totaling 2 mL/well.

On day 3 of culture, cells from the supernatant were collected in 15 mL tubes. Adhering cells were dispersed with Trypsin-EDTA (0.25%) (Thermo Fisher Scientific), and 5-fold amount of medium was added to inactivate Trypsin. The cells were then collected in a 15 mL tube (tube used for supernatant collection).

FCM measurements were performed to determine the ratio of CAR-T cells to cancer cells and the characteristics of CAR-T cells.

The characteristics of CAR-T cells were examined using the same method as described in ("2. FCM measurement"). CAR positivity and PD-1 levels were measured by staining dead cells with 7-AAD (Miltenyi Biotec). 7-AAD-negative cell group and CD3-positive cell group analyzed using FCM. FCM results were analyzed using MACSQuantify^{™} analyzer software.

The ratios were determined by staining CAR-T cells with anti-CD3 antibody and staining cancer cells with anti-CD221 antibody (BioLegend), and analyzing using MACSQuant (registered trademark) analyzer 10 (Miltenyi Biotec).

Antibody staining was performed by adding the manufacturer's specified per-test amount to 1×10⁶ cells/100 µL of D-PBS at 4°C, and reacting for 15 min at 4°C.

Dead cells were stained with 7-AAD (Miltenyi Biotec) and analyzed. FCM results were analyzed using MACSQuantify^{™} analyzer software.

CAR-T cells were prepared and analyzed under the same conditions as in "1. cell preparation" except that the amounts of anti-CD3 antibody and anti-CD28 antibody added to PBMC were changed to 75 ng, 300 ng, and 1000 ng, respectively.

### 4. results etc.

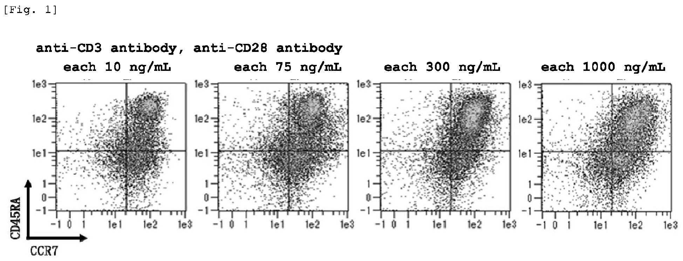

The results of flow cytometry on pre-culture Day 7, CAR-T cell Day 14, and co-culture Day 3 are shown in Table 1. The results of flow cytometry of the cell population at pre-culture Day 7 are shown in Fig. 1.
stimulants (active agent 1-factor 1, anti-CD3 antibody: active agent 2-factor 2, anti-CD28 antibody).
·Tscm: stem cell memory T cells (CD45RA and CCR7 double positive)
·Tcm: central memory T cells (CD45RA-negative, and CCR7-positive)

**[Table 1]**

| pre-culture stimulation | | | pre-culture Day7 | | CAR-T cell Day14 | | | co-culture Day3 E:T=4:1 | |
|---|---|---|---|---|---|---|---|---|---|
| factor 1 | factor 2 | amount added/well | fold expansion | naive/Tscm % | CAR-positive cell count | fold expansion after EP | fold expansion including pre-culture | Rh30% | naive/Tscm % |
| anti-CD3 antibody | anti-CD28 antibody | each 10 ng/ml. | 3.2 | 48.2 | 1.2E+07 | 1.6 | 5.3 | 0.3 | 51.3 |
| anti-CD3 antibody | anti-CD28 antibody | each 75 ng/ml. | 3.4 | 50.9 | 2.5E+07 | 3.4 | 11.5 | NT | NT |
| anti-CD3 antibody | anti-CD28 antibody | each 300 ng/ml. | 5.4 | 67.2 | 3.6E+07 | 4.8 | 26.1 | NT | NT |
| anti-CD3 antibody | anti-CD28 antibody | each 1000 ng/ml. | 8.8 | 54.6 | 3.2E+07 | 4.3 | 37.5 | 0.1 | 37.1 |

From the FCM results, it became clear that a higher stimulation amount (higher amount of stimulant added) reduces the proportion of young cells (naive/Tscm and Tcm) in the obtained CAR-T cells after the anti-cancer assay (after co-culture). This suggests that even with high amounts of stimulation, CAR-T cells can be produced, but they may have an exhausted state.

CAR-T cells were prepared and analyzed under the same conditions as in "1. cell preparation" except that the stimulant added to PBMC was changed to TransAct (Miltenyi Biotec) 1, 3, 30 µL, and similar results were obtained.

CAR-T cells were prepared and analyzed under the same conditions as in "1. cell preparation" except that the PBMCs were changed to PBMCs containing 30%, 50%, or 80% T cells, and similar results were obtained.

### Example 2: culture after T cell isolation and production of CAR-T cells

CAR-T cells were produced after extracting T cells and then pre-culturing them, using cryopreserved peripheral blood mononuclear cells (PBMCs).

### (material and method)

### 1. cell preparation

Anti-CD3 antibody and anti-CD28 antibody (BioLegend) were diluted with D-PBS and seeded onto 48-well plates (Corning) to 10 ng and 1000 ng, respectively, and coated at 37°C for 1 hr.

PBMC (HEM) was thawed in a 37°C water bath, and T cells were isolated using CD4 and CD8 magnetic beads according to the manufacturer's recommended protocol.

The subsequent procedure was the same as in Example 1, except that the cells were seeded onto antibody-coated 48-well plates.

### 2. gene transfer

The procedure was performed by a method similar to that in Example 1, except that the amount of PB transposase plasmid and pIRII-EPHB4-28z plasmid was 2 µL per 300 µL, and the amount of PBMC used for introducing plasmid DNA by electroporation was about 1.5×10⁷ cells.

### 3. expansion culture

The procedure was performed by a method similar to that in Example 1, except that beads were added on day 1 and day 7 after electroporation, and the amount of beads added was 0.25×10⁶.

### (analysis and results)

1. Cell count measurement, 2. flow cytometry (FCM) measurement, and 3. anti-cancer assay were performed by a method similar to that in Example 1.

### 4. results etc.

The results of flow cytometry on pre-culture Day 7, CAR-T cell Day 14, and co-culture Day 3 are shown in Table 2.

**[Table 2]**

| pre-culture stimulation | | | pre-culture Day7 | | CAR-T cell Day14 | | | co-culture Day3 E:T=4:1 | |
|---|---|---|---|---|---|---|---|---|---|
| factor 1 | factor 2 | amount added/well | fold expansion | naive/Tscm % | CAR-positive cell count | fold expansion after EP | fold expansion including pre-culture | Rh30% | naive/Tscm % |
| anti-CD3 antibody | anti-CD28 antibody | each 10 ng/ml. | 5.0 | 59.1 | 3.1E+07 | 3.1 | 15.4 | 0.8 | 27.7 |
| anti-CD3 antibody | anti-CD28 antibody | each 1000 ng/ml. | 4.7 | 59.0 | 3.8E+07 | 4.0 | 18.9 | 39.8 | 20.7 |

From the FCM results, it became clear that a higher stimulation amount (higher amount of stimulant added) reduces the anti-cancer activity of the obtained CAR-T cells, and also reduces the proportion of young cells (naive/Tscm and Tcm) in the obtained CAR-T cells after the anti-cancer assay (after co-culture). This suggests that even with high amounts of stimulation, CAR-T cells can be produced, but they may have an exhausted state.

### Example 3: production of CAR-T cells using virus

CAR-T cells were produced by a viral method, using cryopreserved peripheral blood mononuclear cells (PBMCs).

### (material and method)

### 1. cell preparation

The procedure was performed by a method similar to that in Example 1, except that 10 µL or 60 µL of TransAct (Miltenyi Biotec) was added instead of antibody-conjugated beads.

### 2. gene transfer

The cells were centrifuged by a method similar to that in Example 1, and the obtained cell pellet was suspended in ALySTM705 Medium (Cell Science & Technology Institute). The cells were infected with CD19-CAR lentivirus by suspending and cultured in a 37°C, 5% CO₂ incubator.

### 3. expansion culture

After gene transfer, the cells were cultured for 7 days while adding complete medium (CAR-T cells Day 7). On day 1 after infection, beads conjugated with CD19-CD80-4-1BBL were added, and the cells were expansion cultured.

The culture vessel was prepared by a method similar to that in Example 1. On Day 7, cell count measurement, FCM analysis, and anti-cancer assay were performed.

### (analysis and results)

### 1. Cell count measurement was performed by a method similar to that in Example 1.

### 2. flow cytometry (FCM) measurement

The antibody staining was performed by a method similar to that in Example 1, except that anti-CD19 antibody was used for primary staining.

### 3. anti-cancer assay

Cancer cells used were NALM6 cells (ATCC) expressing CD19.

The assay involved harvesting cells on day 3 of co-culture and subjecting them to analysis.

Furthermore, the anti-cancer assay was repeated six times (total of 18 days) by adding the co-cultured cells, which had been replaced with fresh medium, to NALM6 cells and culturing them for 3 days.

The medium used during co-culture was NALM6 cell medium (RPMI1640-10% FBS medium).

The ratio was determined by staining CAR-T cells with anti-CD3 antibody, staining cancer cells with anti-CD19 antibody (BioLegend), and analyzing using MACSQuant (registered trademark) analyzer 10 (Miltenyi Biotec).

Other methods were performed by a method similar to that in Example 1.

### 4. results etc.

The results of flow cytometry on pre-culture Day 7, CAR-T cell Day 7, and co-culture Day 3 are shown in Table 3.

**[Table 3]**

| pre-culture stimulation | | pre-culture Day7 | | CAR-T cell Day7 | | | co-culture Day3 E:T=4:1 | |
|---|---|---|---|---|---|---|---|---|
| stimulant | amount added/well | fold expansion | naive/Tscm % | CAR-positive cell count | fold expansion after infection | fold expansion including pre-culture | NALM6% | naive/Tscm % |
| TransAct | 10 µL | 13.9 | 57.9 | 3.82E+07 | 17.6 | 244.6 | 0.0 | 28.3 |
| TransAct | 60 µL | 13.9 | 51.3 | 6.50E+07 | 34.2 | 475.4 | 0.0 | 37.6 |

From the FCM results, cells with good proliferation and good anti-cancer activity were obtained regardless of whether the TransAct amount during pre-culture was 10 µL or 60 µL. Furthermore, it was clarified that even after co-culture, the cells were of high quality, with a naive/Tscm ratio of 20% or more.

The CAR-T cells produced completely eliminated NALM6 cells even after the second to sixth co-culture, demonstrating the production of cells with persistence.

### [Industrial Applicability]

According to the method of the present invention, naive and/or memory T cells can be maintained and proliferated, and a sufficient amount of T cells suitable for T cell therapy and CAR-T cell therapy can be obtained.

This application is based on a patent application No. 2023-221979 filed in Japan (filing date: December 27, 2023), the contents of which are incorporated in full herein.

## Claims

1. A method for culturing a T cell, comprising a step of contacting a composition comprising T cells with a stimulant, wherein
the stimulant activates (i) one or more intracellular signaling domains of one or more components of a TCR complex and (ii) one or more intracellular signaling domains of one or more co-stimulatory molecules,
an amount of the stimulant added is 1 to 1500 ng per 10⁶ cells in the composition comprising T cells, and
a proportion of T cells in the composition comprising T cells is 5% or more of the total number of cells in the composition.

2. A method for culturing a T cell, comprising a step of contacting a composition comprising T cells with a stimulant, wherein
the stimulant activates (i) one or more intracellular signaling domains of one or more components of a TCR complex and (ii) one or more intracellular signaling domains of one or more co-stimulatory molecules, and
an amount of the stimulant added is 1 to 1500 ng per 10⁶ T cells in the composition comprising T cells.

3. The method according to claim 1 or 2, comprising a step of separating T cells from the composition comprising T cells.

4. The method according to claim 1 or 2, wherein the method for culturing T cells is a method for maintaining or proliferating T cells.

5. The method according to claim 1 or 2, wherein the T cell is a naive and/or memory T cell.

6. The method according to claim 1 or 2, wherein the stimulant comprises an anti-CD3 antibody or an antigen-binding fragment thereof and an anti-CD28 antibody or an antigen-binding fragment thereof.

7. The method according to claim 6, wherein a ratio of the anti-CD3 antibody or an antigen-binding fragment thereof to the anti-CD28 antibody or an antigen-binding fragment thereof contained in the stimulant is 1:0.5 to 1:200.

8. The method according to claim 1 or 2, wherein the stimulant is present on a support.

9. The method according to claim 8, wherein the support is a bead or a nanomatrix.

10. A method for producing a chimeric antigen receptor T (CAR-T) cell, comprising the following steps:
(1) a step of culturing T cells by the method according to claim 1 or 2 (pre-culture step),
(2) a step of introducing the CAR gene into the pre-cultured T cells (CAR-T introduction step), and when desired,
(3) a step of expanding the cells into which the CAR gene has been introduced (expansion culture step).

11. An additive for T cell culture, comprising an anti-CD3 antibody or an antigen-binding fragment thereof and an anti-CD28 antibody or an antigen-binding fragment thereof, wherein the T cell is naive and/or memory T cell.

12. The additive according to claim 11, which is used in a dose of 1 to 1500 ng per 10⁶ T cells.
